Europäisches Patentamt

European Patent Office

Office européen des brevets

(19)

(11) Veröffentlichungsnummer: **0 077 034**
**B1**

(12) **EUROPÄISCHE PATENTSCHRIFT**

(45) Veröffentlichungstag der Patentschrift:
**02.05.85**

(51) Int. Cl.⁴: **A 61 L 15/01,** A 61 L 15/03,
A 61 F 13/00

(21) Anmeldenummer: **82109300.2**

(22) Anmeldetag: **07.10.82**

(54) **Saugfähige Wundauflage.**

(30) Priorität: **09.10.81 DE 8129563 U**

(43) Veröffentlichungstag der Anmeldung:
**20.04.83 Patentblatt 83/16**

(45) Bekanntmachung des Hinweises auf die Patenterteilung:
**02.05.85 Patentblatt 85/18**

(84) Benannte Vertragsstaaten:
**AT BE CH DE FR GB LI LU NL SE**

(56) Entgegenhaltungen:
**AT - B - 310 951
DE - A - 1 642 025
DE - B - 1 642 082
US - A - 4 203 435**

(73) Patentinhaber: **Rauscher & Co. Verbandstoff- und Wattefabriken, Johann Schorsch Gasse 4, A-1141 Wien (AT)**

(72) Erfinder: **Leuprecht, Helmut, Dr., Sankt Veitgasse 26, A-1130 Wien (AT)**

(74) Vertreter: **Patentanwälte Leinweber & Zimmermann, Rosental 7/II Aufg., D-8000 München 2 (DE)**

## Beschreibung

Die Erfindung betrifft eine saugfähige Wundauflage, bestehend aus einem Kern hoch saugfähigem Materials in einer Hüllschicht, deren wundseitige Aussenschicht Gitterstruktur aufweist und aus mit der Wunde nicht verklebenden Material besteht.

Bekannte Wundauflagen dieser Art (AT-S Nr. 310951) werden dadurch hergestellt, dass man eine Zellstoffschicht aussen mit einer unzusammenhängenden haftungsfeindlichen Acrylharzschicht überzieht. Dieser können auch keimtötende Wirkstoffe zugesetzt werden. Herstellen lässt sich ein solches Produkt nur dadurch, dass die Zellstoffschicht durch ein Acrylharztauchbad geführt wird. Damit wird die Zellstoffschicht aber auch an ihren inneren Oberflächen mit Acrylharz imprägniert und verliert so ihre Saugfähigkeit. Auch ist die Ausbildung der Gitterstruktur der Acrylharzschicht selbst nur schwer sicherzustellen. In der Regel wird deshalb die Hüllschicht auf einem Prägezylinder nachzuprägen sein, um die Poren für den Durchtritt des Wundsekretes sicherzustellen.

Als Wundauflage schon bekannt sind dünne Zweischichtenvliese (DE-OS Nr. 1642025), bei denen jede der beiden Schichten aus Fasern besteht. Die wundseitige Aussenschicht wird dabei aus Polyäthylen- oder Polypropylenfasern gebildet, die mit der Wunde nicht verkleben, aber auch keine Aufnahmefähigkeit für Sekrete haben. Die darunterliegende Schicht besteht aus Viskose- oder Baumwollfasern, die die notwendige Saugfähigkeit für den Sekretabtransport aufweist. Derartige Faserschichten haben eine gewisse Mindeststärke. Die saugfähigen Schichten können deshalb nur bis zur Mindeststärke der nicht saugfähigen Aussenschicht an die Wunde herangebracht werden. Die aus Fasern aufgebaute Aussenschicht hat aber ihrer Grösse nach schwer zu steuernde, meist zu kleine Öffnungen. Es kommt hier rasch zu einem Zusetzen dieser Öffnungen durch trocknendes Sekret. Der weitere Abtransport des Sekretes in die saugfähigen Lagen ist dann unterbrochen. Das ist ungünstig.

Schliesslich ist es auch schon bekannt, die Oberfläche von mehrschichtigen Wundauflagen aus einer perforierten Polypropylenfolie zu bilden. Diese lässt sich zwar ebenfalls gut von einer Wunde abnehmen, da sie nicht mit ihr verklebt. Die zwischen den Perforationen verbleibenden Flächenbereiche der Folie sind aber völlig undurchlässig. Der Abtransport des Sekretes aus diesen Bereichen durch die Perforation ist erschwert. Die Kanalisation des Sekrets durch die Perforationen überfordert diese häufig. Das Sekret kommt zum Stehen und erstarrt. Der Sekretabtransport ist dann völlig unterbrochen.

Aufgabe der Neuerung ist es, eine Wundauflage, die aus einem Kern hoch saugfähigen Materials in einer Hüllschicht besteht, deren wundseitige Aussenschicht aus mit der Wunde nicht verklebendem Material Gitterstruktur aufweist, so auszugestalten, dass bei Beibehaltung der Verhinderung des Verklebens mit der Wunde der Sekretabtransport in den saugfähigen Kern der Wundauflage sichergestellt und der Heilerfolg gegebenenfalls noch durch Imprägnierung mit antibakteriellen Substanzen unterstützt wird.

Diese Aufgabe wird erfindungsgemäss dadurch gelöst, dass wundseitig eine zweischichtige Hüllschicht aus einem kernseitig angeordneten heisssiegelfähigen Faservlies vorgesehen wird, das auf ein aussenliegendes Polypropylengewebe mit Gitterstruktur aufkaschiert ist.

Die Aussenschicht ist also weder eine Folie, noch eine Vliesschicht, sondern ein Polypropylengewebe. Das Material stellt sicher, dass das Verkleben mit der Wunde vermieden wird. Der Aufbau als Polypropylengewebe ermöglicht es auf einfache Weise, genügend grosse Öffnungen der Gitterstruktur als Sekretkanäle zu schaffen. Durch die Webstruktur des Polypropylengewebes ist die Berandung dieser Sekretkanäle überdies uneben und unregelmässig, was einem Sekretstau entgegenwirkt und den Sekretabtransport begünstigt. Die Festigkeit des Polypropylengewebes ist auch bei sehr geringer Schichtstärke und relativ hohem Anteil an die Schicht durchbrechenden Öffnungen an der Gesamtfläche fest und stabil. Von entscheidender Bedeutung ist überdies die Hinterlegung des Polypropylengewebes auf der wundabgewandten Seite mit dem Faservlies. Dieses ist durch seine Heisssiegelfähigkeit unmittelbar mit dem Gewebe verbunden und damit sehr dicht an die Wunde herangebracht. In den von der Gitterstruktur des Polypropylengewebes gebildeten Öffnungen liegt es für die Wundsekrete frei. Diese werden deshalb bei ihrem Eintritt in die Sekretkanäle sofort vom Faservlies aufgenommen und in das Innere der Wundauflage abtransportiert, deren Kern aus hoch saugfähigem Material besteht. Wichtig hierfür ist es, dass das auf das Polypropylengewebe auf der von der Wunde abgewandten Seite aufkaschierte Faservlies nicht lose und im unterschiedlichen Abstand vom Kunstfasergewebe zu liegen kommt, sondern mit diesem einen echten Zweischichtenverbund bildet. Um dies zu erreichen, wird gerade das heisssiegelfähige Faservlies verwendet und auf das Kunstfasergewebe aufkaschiert.

Vorteilhaft werden dabei die beiden Schichten der zweischichtigen Hüllschicht im wesentlichen flächig miteinander verbunden. Es müssen also zumindest über die ganze Fläche dicht verteilte Verbindungspunkte zwischen den Schichten vorliegen. Ist das der Fall, so können aus der Wundauflage je nach Bedarf Stücke beliebiger Grösse hergestellt werden. Diese behalten dann die oben erläuterte Struktur auch unabhängig vom Zuschnitt und lösen sich nicht in ihre Einzelbestandteile auf. Dies stellt für die Verarbeitung und bei der Anwendung einen wesentlichen Vorteil dar. Auch die Vorratshaltung ist vereinfacht, weil nicht zu viele unterschiedliche Grössen erforderlich sind.

Besonders günstig ist es, wenn die oben erläuterte zweischichtige Hüllschicht nur wundseitig vorgesehen ist, auf der von der Wunde abliegenden Aussenseite der Wundauflage jedoch nur eine einschichtige Hüllschicht aus dem Faservlies vorgesehen ist.

Der Aufbau der zweischichtigen Hüllschicht aus dem Polypropylengewebe mit aufkaschiertem heisssiegelfähigen Faservlies erlaubt auch eine besonders günstige Ausrüstung mit den Heilprozess unterstützenden Wirkstoffen. Es kann nämlich die zweischichtige Hüllschicht wundseitig mit einer Salbschicht versehen und sich in Richtung auf den saugfähigen Kern, also auf ihrer von der Wunde abgewandten Seite mit einer Hemmschicht hinterlegt sein, die sich dem Durchtritt der Salbe in den saugfähigen Kern widersetzt. Bei der Salbschicht kann es sich zweckmässig um eine antibakterielle PVP-Jodsalbe handeln. Als Hemmschicht ist ein Polypropylenvlies geeignet. Die Hemmschicht entfaltet ihre den Durchtritt in den saugfähigen Kern verhindernde Wirkung nur für ein Material mit hoher Viskosität, wie es die Salbe darstellt, Material mit niedriger Viskosität, wie die Wundsekrete, werden von ihr jedoch relativ ungehindert durchgelassen. Der beschriebene Mehrschichtenaufbau ist deshalb nach der Herstellung bis zur Verwendung in einem statisch stabilen Zustand, bei dem die Schichtstruktur des erfindungswesentlichen Vierschichtaufbaus Salbschicht, Polypropylengewebe, aufkaschiertes Faservlies, Hemmschicht weitgehend aufrechterhalten wird. Nach Auflage auf die Wunde tritt eine doppelte Wirkung ein: Das Sekret und die steigende Temperatur setzen die Viskosität der Salbe herab. Deren Wirkstoffe gelangen so an die Wunde. Andererseits wird aber das Wundsekret nach wie vor in den hoch saugfähigen Kern abtransportiert, da für das Sekret mit niedriger Viskosität die Hemmschicht nicht wirksam ist.

Bei Verwendung eines selbstzusammenhaltenden Kernmaterials, wie Zellstoff oder Watte, wird die zweischichtige Hüllschicht zweckmässig schlauchartig mit offenen Enden aufgebaut. Bei Verwendung eines losen Kernmaterials, wie Pulpe, muss die Hüllschicht jedoch allseitig verschlossen sein. Zur Herstellungsvereinfachung kann man auch in diesem Fall die Hüllschicht schlauchartig aufbauen und an ihren offenen Enden versiegeln.

Speziell bei der mit der Salbschicht aufgebauten Wundauflage ist es günstig, die Wundauflage insgesamt in eine allseits verschlossene Packhülle einzusiegeln, in der sie sterilgehalten ist. Besonders günstig als Packhülle ist eine Aluminiumfolie, die Temperaturbelastungen der Wundauflage vor ihrem Einsatz vermindert. Im folgenden wird die Erfindung anhand von Beispielen erläutert.

*Beispiel 1:*

Ein vorfabriziertes Polypropylengewebe aus im Querschnitt runden, gegebenenfalls aber auch flachen Polypropylenfäden wird von einer Vorratsrolle abgezogen, von einer anderen Vorratsrolle wird ein heisssiegelfähiges Faservlies *(nonwoven)* abgezogen, bei dem es sich beispielsweise um ein Zellwollvlies handelt. Die beiden Bahnen des Polypropylengewebes und des heisssiegelfähigen Faservlieses werden sodann über eine Umlenkwalze zusammengeführt und aufeinanderliegend einem Kalanderwerk zugeführt; hier werden die beiden Bahnen übereinanderliegend durch den Spalt zwischen zwei Kalanderwalzen durchgeführt, von denen eine auf eine Temperatur von ca. 200°C oberflächlich aufgeheizt ist. Durch die Temperatureinwirkung wird das Faservlies auf das Polypropylengewebe aufkaschiert. Die Verbindung kann dabei je nach der Struktur der Kalanderwalzen (glatt, gerillt, punktförmig usw.) flächig, streifenförmig, punktförmig usw. erzielt werden. Die auf diese Weise hergestellte Hüllschicht wird dann um einen Kern aus saugfähigem Material aus Zellstoff, Watte, Pulpe oder dergleichen herumgeschlagen. Dabei überlappen sich die beiden Längsränder auf der von der Wunde abliegenden Seite. Die Schmalränder werden verprägt.

*Beispiel 2:*

Von einer Endlosbahn des Kernmaterials werden vorzugsweise rechteckige Stücke abgeschnitten und auf einem Tragband mit vorbestimmten Abstand unereinander abgelegt. Die zweischichtige Hüllschicht wird hergestellt, wie oben im Beispiel 1 beschrieben. Nach dem Aufkaschieren des Faservlieses auf das Polypropylengewebe wird die Bahn zwischen Tragband und Stücke des Kernmaterials eingeführt und auf der wundabgewandten Seite zu einem Schlauch verschweisst. Anschliessend wird der Schlauch im Bereich zwischen den Stücken des Kernmaterials verprägt oder versiegelt und anschliessend in diesem Bereich so geschnitten, dass man allseits umhüllte und versiegelte Wundauflagen erhält.

*Beispiel 3:*

Bei der Herstellung der Hüllschicht nach Beispiel 1 wird mit Bahnen unterschiedlicher Breite gearbeitet: Das Polypropylengewebe hat dabei nur eine geringfügig grössere Breite als die fertige Wundauflage. Das Faservlies wird jedoch erheblich breiter gewählt und erhält eine Breite, die das Doppelte der fertigen Wundauflage übersteigt. Bei dem Zusammenführen der beiden Bahnen für das Aufkaschieren des Faservlieses auf das Polypropylengewebe werden die beiden Bahnen so zusammengeführt, dass ihre Längsmittelachsen etwas gegeneinander versetzt sind. Dadurch ist der Überstandsrand des Faservlieses auf der einen Seite grösser als auf der anderen Seite. Die entstehende zweischichtige Hüllschicht wird sodann ähnlich wie im Beispiel 2 weiterverarbeitet. Bei der fertigen Wundauflage greifen dabei die Seitenränder des Polypropylengewebes nur geringfügig um die Seitenränder der fertigen Wundauflage auf deren Rückseite herum. Die unterschiedlich breiten Faservliesüberstände ermöglichen ein einfaches Heisssiegeln und damit Verschliessen auf der Rückseite.

*Beispiel 4:*

Aus Polypropylenfasern wird auf übliche Weise ein Polypropylenvlies hergestellt, das als Hemmschicht dienen soll. Dieses kann auf einer Oberfläche einer Wärmebehandlung unterworfen werden, um so eine den Durchtritt der zähen Salbmasse weiter erschwerende Oberflächenschicht zu erzielen. Die so entstandene Schicht wird dann mit einer Bahn hoch saugfähigen Kernmaterials unterlegt. Diese Bahn weist zweckmässig eine hohe Stärke auf, um eine gute Abpolsterung und eine hohe Saugkapazität sicherzustellen. Der entstandene Schichtverbund aus dem hoch saugfähigen Kernmaterial und dem als Hemmschicht dienenden Polypropylenvlies (von unten nach oben) wird dann entsprechend den Erläuterungen in den obigen Beispielen mit der zweischichtigen Hüllschicht umgeben. Die entstandene mehrschichtige Endlosbahn wird sodann mechanisch unterstützt unter einer Düse durchgeführt, mit deren Hilfe der wundseitige Salbauftrag zur Ausbildung der Salbschicht erfolgt. Anschliessend erfolgt die Auftrennung in einzelne Stücke durch Schneiden.

*Beispiel 5:*

Die nach den obigen Beispielen 1 bis 4 hergestellte Wundauflage wird anschliessend in einem zusätzlichen Arbeitsgang mit einer aus Endlosfolie hergestellten Packhülle umgeben, die in der gewünschten Grösse abgeschnitten und heissgesiegelt, verschweisst oder auf andere Weise hermetisch verschlossen wird. Die Packhülle besteht zweckmässig aus einer Aluminiumfolie, die auf ihrer Innenseite der Wundauflage zugewendet eine antiädhasive und luftundurchlässige Beschichtung, zweckmässig aus einem dünnen Polypropylenfilm, aufweisen kann.

## Patentansprüche

1. Saugfähige Wundauflage, bestehend aus einem Kern hoch saugfähigen Materials in einer Hüllschicht, deren wundseitige Aussenschicht Gitterstruktur aufweist und aus mit der Wunde nicht verklebendem Material besteht, dadurch gekennzeichnet, dass wundseitig eine zweischichtige Hüllschicht aus einem kernseitig angeordneten heisssiegelfähigen Faservlies vorgesehen ist, das auf ein aussenliegendes Polypropylengewebe mit Gitterstruktur aufkaschiert ist.

2. Wundauflage nach Anspruch 1, dadurch gekennzeichnet, dass in der zweischichtigen Hüllschicht das heisssiegelfähige Faservlies mit dem Gitterstruktur aufweisenden Polypropylengewebe grossflächig verbunden ist.

3. Wundauflage nach Anspruch 1, dadurch gekennzeichnet, dass auf der von der Wunde abliegenden Aussenseite eine einschichtige Hüllschicht aus dem Faservlies vorgesehen ist.

4. Wundauflage nach Anspruch 1, dadurch gekennzeichnet, dass zusätzlich auf der zweischichtigen Hüllschicht wundseitig eine die Aussenlage bildende Salbschicht aufgebracht sowie zwischen die zweischichtige Hüllschicht und den saugfähigen Kern eine sich dem Durchtritt der Salbe in den saugfähigen Kern widersetzende Hemmschicht eingebracht ist.

5. Wundauflage nach Anspruch 4, dadurch gekennzeichnet, dass die Salbschicht aus einer antibakteriellen PVP-Jodsalbe besteht.

6. Wundauflage nach Anspruch 5, dadurch gekennzeichnet, dass die Hemmschicht aus einem Polypropylenvlies besteht.

7. Wundauflage nach Anspruch 1, dadurch gekennzeichnet, dass die zweischichtige Hüllschicht schlauchartig mit offenen Enden ausgebaut ist.

8. Wundauflage nach Anspruch 7, dadurch gekennzeichnet, dass die schlauchartig ausgebaute zweischichtige Hüllschicht an ihren offenen Enden versiegelt ist.

9. Wundauflage nach einem der Ansprüche 1 oder 4, dadurch gekennzeichnet, dass die Wundauflage in eine allseits verschlossene Packhülle eingesiegelt ist.

10. Wundauflage nach Anspruch 9, dadurch gekennzeichnet, dass die Packhülle aus einer allseits versiegelten, auf ihrer Innenseite gegebenenfalls und zwar vorzugsweise mit einem Kunststofffilm beschichteten Aluminiumfolie besteht.

## Revendications

1. Pansement absorbant pour blessures, constitué par un cœur en matériau à pouvoir absorbant élevé dans une enveloppe dont la couche extérieure côté blessure présente une structure réticulée et est faite en matériau ne collant pas à la blessure, caractérisé en ce qu'il est prévu du côté blessure une enveloppe à deux couches faite d'une nappe de fibres thermosoudable disposée du côté du cœur, qui est contrecollée sur un tissu de polypropylène à structure réticulée disposé à l'extérieur.

2. Pansement pour blessures selon la revendication 1, caractérisé en ce que, dans l'enveloppe à deux couches, la nappe de fibres thermosoudable est assemblée par grande surface avec le tissu de polypropylène possédant une structure réticulée.

3. Pansement pour blessures selon la revendication 1, caractérisé en ce que, sur le côté extérieur situé à l'opposé de la blessure, il est prévu une enveloppe à une seule couche faite avec la nappe de fibres.

4. Pansement pour blessures selon la revendication 1, caractérisé en ce que, en supplément, est appliquée sur l'enveloppe à deux couches, du côté blessure, une couche d'onguent formant la couche extérieure, et qu'il est intercalé entre l'enveloppe à deux couches et le cœur absorbant une couche d'arrêt s'opposant à la pénétration de l'onguent dans le cœur absorbant.

5. Pansement pour blessures selon la revendication 4, caractérisé en ce que la couche d'onguent est faite d'une pommade iodée au PVP bactéricide.

6. Pansement pour blessures selon la revendication 5, caractérisée en ce que la couche d'arrêt est constituée par une nappe de fibres de polypropylène.

7. Pansement pour blessures selon la revendication 1, caractérisé en ce que l'enveloppe à deux couches est en forme de gaine avec extrémités ouvertes.

8. Pansement pour blessures selon la revendication 7, caractérisé en ce que l'enveloppe à deux couches en forme de gaine est scellée à ses extrémités ouvertes.

9. Pansement pour blessures selon l'une des revendications 1 ou 4, caractérisé en ce que le pansement est scellé dans un emballage fermé de tous les côtés.

10. Pansement pour blessures selon la revendication 9, caractérisé en ce que l'emballage est constitué par une feuille d'aluminium scellée de tous les côtés, possédant éventuellement sur sa face inférieure un revêtement qui est de préférence un film de matière synthétique.

## Claims

1. Absorbent wound dressing comprising a core of highly absorbent material within an envelope layer whose wound-side outer layer has a lattice structure and consists of a material which does not stick to the wound, characterised in that at the wound side there is provided a two-ply envelope layer comprising a heat-sealable fibre fleece which is arranged at the core side and which is laminated onto an externally situated polypropylene fabric having a lattice structure.

2. Wound dressing according to Claim 1, characterised in that in the two-ply envelope layer the heat-sealable fibre fleece is connected over large areas to the lattice-structure polypropylene fabric.

3. Wound dressing according to Claim 1, characterised in that at the outer side remote from the wound there is provided a single-ply envelope layer consisting of the fibre fleece.

4. Wound dressing according to Claim 1, characterised in that there is additionally applied to the two-ply envelope layer, at the wound side, an ointment layer forming the outside layer, and also an inhibiting layer opposing the passage of the ointment through into the absorbent core is introduced between the two-ply envelope layer and the absorbent core.

5. Wound dressing according to Claim 4, characterised in that the ointment layer consists of an anti-bacterial PVP iodine ointment.

6. Wound dressing according to Claim 5, characterised in that the inhibiting layer consists of a polypropylene fleece.

7. Wound dressing according to Claim 1, characterised in that the two-ply envelope layer is made in tubular form with open ends.

8. Wound dressing according to Claim 7, characterised in that the two-ply envelope layer made in a tubular form is sealed at its open ends.

9. Wound dressing according to one of Claims 1 or 4, characterised in that the wound dressing is sealed into an envelope pack closed on all sides.

10. Wound dressing according to Claim 9, characterised in that the envelope pack consists of an aluminium foil which is sealed all round and which may be, and in fact preferably is, coated internally with a film of synthetic plastic material.